# EUROPEAN PATENT SPECIFICATION

(11) **EP 2 663 269 B1**
(45) Date of publication and mention of the grant of the patent: **31.12.2014**
(21) Application number: 12702055.0
(22) Date of filing: 13.01.2012
(51) Int. Cl.: A61F 5/44

(54) **OSTOMY BAG**
OSTOMIEBEUTEL
SAC D'OSTOMIE

(30) Priority: 14.01.2011 GB 201100609
(43) Date of publication of application: 20.11.2013
(73) Proprietor: WELLAND MEDICAL LIMITED, Crawley, West Sussex RH10 2TU (GB)
(72) Inventor: BIRD, Paul, West Sussex RH10 2TU (GB); FINN, Richard, West Sussex RH10 2TU (GB)
(74) Representative: Lock, Graham James
(86) International application number: PCT/GB2012/000033
(87) International publication number: WO 2012/095638

(56) References cited:
- WO-A2-2005/082271
- GB-A- 2 394 897
- GB-A- 2 399 755
- US-A- 6 135 986

## Description

The present invention relates to ostomy bags for collection of solid waste from a human or animal body. In particular the invention provides an ostomy bag with specific design features addressing the problem of "pancaking". Document WO 2005/0822721 A is regarded as the closest prior art.

Pancaking is a term used to describe the situation where large quantities of soft waste are deposited with speed to the inlet of a clean ostomy bag, they stick to the immediately opposing inner surface of the bag, blocking the inlet and preventing use of space distanced from the outlet. As a consequence of pancaking, despite there being available capacity in the bag, the bag can overflow when subsequent deposits are attempted.

The present invention seeks to provide an improved ostomy bag which substantially reduces the occurrence of pancaking.

In accordance with the present invention, there is provided an ostomy bag having an inlet leading to a waste collection space defined by two opposing surfaces, the opposing surfaces configured to separate on the introduction of waste through the inlet and including a leaf having a contact surface which, when the bag is in an unused configuration, faces the inlet and in use, when an initial deposit is made through inlet collects the deposit and under the weight of the collected deposit drops into the bag thereby separating the opposing surfaces and opening the collection space for receipt of further deposits.

Conveniently, an end of the leaf may be welded into a seam joining the two opposing surfaces at a position distal to the location of the inlet. The leaf is dimensioned to extend over and across the inlet so as to be the first point of contact of an initial deposit into the bag. As a deposit is made the bag begins to separate and the deposit collects on the immediately facing surface of the leaf. With a small space opened between the opposing surfaces of the bag, the leaf falls between the opposing surfaces under the weight of the deposit dragging the deposit into the collection space and holding the space open to receive further deposits.

The leaf is made from a lightweight and flexible material. For example, but without limitation, the leaf comprises a film of polyvinyl acetate, EVA, polyethylene or like polymeric film or a suitable non woven fabric for instance a polyethylene based fabric). The surface area of the leaf is desirably between about 60% to 90% of the surface area of an opposing surface. Conveniently, but not essentially, the leaf has a perimeter shape and size broadly similar to that of the bag but with a margin removed.

In one option, in addition, the surface facing the inlet is provided with a pleat whereby to provide for further expansion of the collection space as additional deposits are received into the bag. The pleat may be a single or double pleat or box pleat.

The walls of the collection bag may comprise any conventional structure known in the prior art. In one preferred option, the bag comprises a laminate which has hydrophobic exposed surfaces and degradable core and means for delaminating the bag whereby to expose the degradable core and facilitate easy flushing of the bag after use.

In an alternative option, the bag comprises a disposable or degradable liner enclosed in a hydrophobic bag and means for conveniently removing the liner for disposal.

In another option the bag may also incorporate an additional feature to prevent ballooning of the bag. The surface of the bag is provided with a closable exit aperture and means for selectively releasing accumulated gas through the aperture. The exit can conveniently be resealably opened by a closure which covers the aperture and attaches to the bag by means of a peelable adhesive. The closure may conveniently be provided in the form of a sealing flap which folds down from a seam around the edge of the bag. Alternatively, the closure may comprise a patch which can be completely detached from the bag and optionally replaced with another. Alternative resealable means to a peelable adhesive will no doubt occur to the skilled addressee and include, without limitation, a mechanical seal comprising an annular rib received in an annular groove or a suction based closure means.

One embodiment of a bag in accordance with the invention is now described with reference to the accompanying figures of which;
Figure 1 shows a face on view of a transparent wall of an unused colostomy bag in accordance with the invention.
Figure 2 shows in stages a), b) and c) how the bag responds to an initial deposit through the inlet.

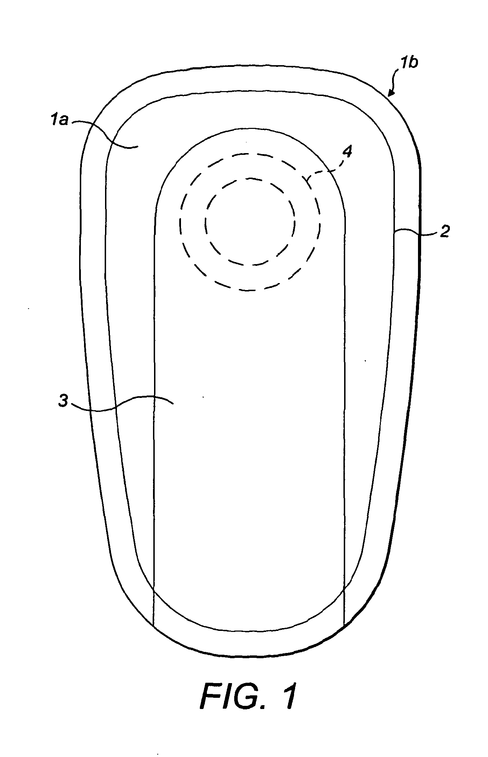

As can be seen from Figure 1 an ostomy bag comprises two opposing walls 1a, 1b defining internally opposing surfaces. The walls are welded together along a seam 2. The front facing wall 1a is transparent and through it can be seen a leaf 3 of flexible lightweight material which is welded into the seam 2 between the two walls 1a, 1b. The dotted outline represents the position of an inlet 4 passing through the rear wall 1b into which, in use, deposits are received. In use, the inlet 4 is positioned above the section of the seam 2 where the leaf is welded between the walls 1a, 1b.

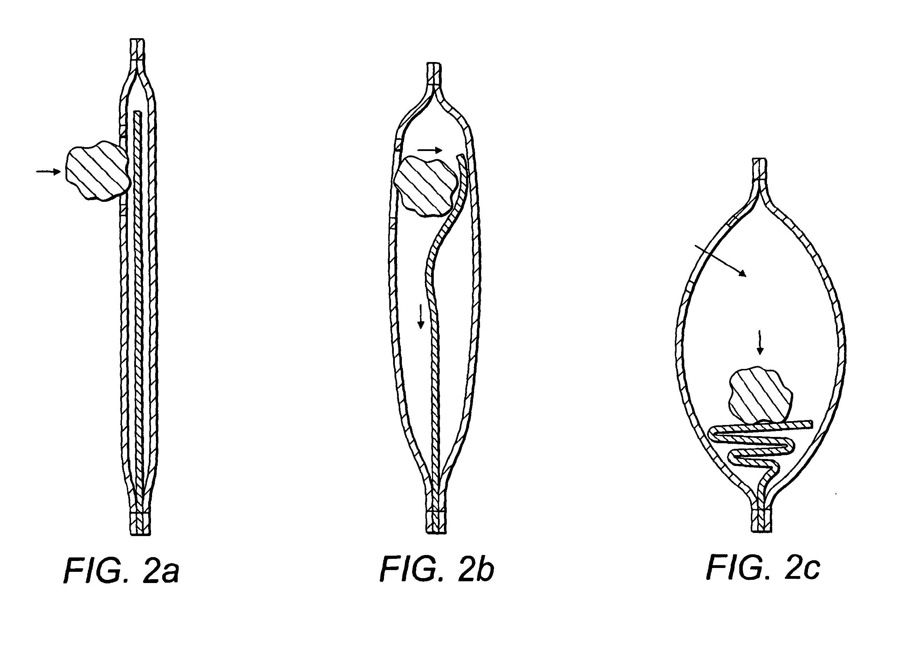

Figure 2 shows in cross section the embodiment of Figure 1 in a side view during three stages. In the first stage, a deposit 5 enters the inlet 4 and immediately interfaces with the leaf 3. (Figure 2a).

Introduction of the deposit forces the walls 1a, 1b apart opening the collection space. No longer sandwiched by the walls, the leaf 3 begins to collapse (Figure 2b).

As the leaf 3 collapses, under gravity, it carries the deposit 5 to the bottom of the collection space where it serves to hold the walls 1a, 1b apart ensuring that optimum use is made of the collection space available. (Figure 2c).

The walls may be of any known or suitable construction, the improvement comprises the introduction of the leaf 3 which can be used to good effect in addressing the problem of pancaking irrespective of the bag construction.

## Claims

1. An ostomy bag having an inlet leading to a waste collection space defined by two opposing surfaces, the opposing surfaces configured to separate on the introduction of waste through the inlet, **characterized in** by including a leaf having a contact surface which, when the bag is in an unused configuration, faces the inlet and in use, when an initial deposit is made through inlet collects the deposit and under the weight of the collected deposit drops into the bag thereby separating the opposing surfaces and opening the collection space for receipt of further deposits.

2. An ostomy bag as claimed in claim 1 wherein an end of the leaf is welded into a seam joining the two opposing surfaces at a position distal to the location of the inlet.

3. An ostomy bag as claimed in claim 1 or claim 2 wherein the leaf is dimensioned to extend over and across the inlet so as to be the first point of contact of an initial deposit into the bag.

4. An ostomy bag as claimed in any preceding claim wherein the leaf is made from a lightweight and flexible material.

5. An ostomy bag as claimed in claim 4 wherein the leaf comprises a film selected from; polyvinyl acetate EVA or polyethylene.

6. An ostomy bag as claimed in claim 4 wherein the leaf comprises a suitable non woven fabric.

7. An ostomy bag as claimed in any preceding claim wherein the surface area of the leaf is between about 60% to 90% of the surface area of an opposing surface.

8. An ostomy bag as claimed in any preceding claim wherein the leaf has a perimeter shape and size broadly similar to that of the bag but with a margin removed.

9. An ostomy bag as claimed in any preceding claim wherein the surface facing the inlet is provided with a pleat whereby to provide for further expansion of the collection space as additional deposits are received into the bag.

10. An ostomy bag as claimed in claim 8 wherein the pleat is selected from a single pleat, a double pleat or a box pleat.

11. An ostomy bag as claimed in any preceding claim wherein the bag comprises a laminate which has hydrophobic exposed surfaces and degradable core and means for delaminating the bag whereby to expose the degradable core and facilitate easy flushing of the bag after use.

12. An ostomy bag as claimed in any of claims 1 to 9 wherein the bag comprises a disposable or degradable liner enclosed in a hydrophobic bag and means for conveniently removing the liner for disposal.

13. An ostomy bag as claimed in any preceding claim further including a closable exit aperture and means for selectively releasing accumulated gas through the aperture.

## Patentansprüche

1. Stomabeutel mit einem Einlass, der zu einem Ausscheidungssammelraum führt, welcher durch zwei gegenüberliegende Flächen definiert ist, wobei die gegenüberliegenden Flächen eingerichtet sind, bei der Einleitung von Ausscheidungen durch den Einlass auseinanderzugehen, **gekennzeichnet durch** das Aufweisen eines Folienelements mit einer Kontaktfläche, die, wenn der Beutel in einer unbenutzten Konfiguration ist, dem Einlass zugewendet ist und bei Verwendung, wenn eine erste Abscheidung **durch** den Einlass erfolgt, die Abscheidung aufnimmt und unter dem Gewicht der aufgenommen Abscheidung sich in den Beutel absenkt, wodurch die gegenüberliegenden Flächen auseinandergehen und es den Sammelraum für die Aufnahme weiterer Abscheidungen öffnet.

2. Stomabeutel nach Anspruch 1, wobei ein Ende des Folienelements in eine Naht, welche die zwei gegenüberliegenden Flächen verbindet, an einer Position distal zu der Einbaustelle des Einlasses eingeschweißt ist.

3. Stomabeutel nach Anspruch 1 oder Anspruch 2, wobei das Folienelement dimensioniert ist, dass es sich über den Einlass hinaus und quer darüber erstreckt, sodass es den ersten Berührungspunkt für eine erste Abscheidung in den Beutel ist.

4. Stomabeutel nach einem der vorhergehenden Ansprüche, wobei das Folienelement aus einem leichtgewichtigen und elastischen Material hergestellt ist.

5. Stomabeutel nach Anspruch 4, wobei das Folienelement eine Folie umfasst, die aus Polyvinylacetat, EVA oder Polyethylen ausgewählt ist.

6. Stomabeutel nach Anspruch 4, wobei das Folienelement einen geeigneten Vliesstoff umfasst.

7. Stomabeutel nach einem der vorhergehenden Ansprüche, wobei der Oberflächenbereich des Folienelements zwischen ungefähr 60 % bis 90 % des Oberflächenbereiches einer gegenüberliegenden Fläche liegt.

8. Stomabeutel nach einem der vorhergehenden Ansprüche, wobei das Folienelement eine Umfangsform und eine Größe aufweist, die weitgehend gleich derjenigen des Beutels ist, allerdings um einen Randstreifen verkleinert.

9. Stomabeutel nach einem der vorhergehenden Ansprüche, wobei die Fläche, welche dem Einlass zugewendet ist, mit einer Falte versehen ist, wodurch eine weitere Ausdehnung des Sammelraums vorgesehen ist, wenn weitere Abscheidungen in dem Beutel aufgenommen werden.

10. Stomabeutel nach Anspruch 8, wobei die Falte aus einer einfachen Falte, einer doppelten Falte oder einer Kellerfalte ausgewählt ist.

11. Stomabeutel nach einem der vorhergehenden Ansprüche, wobei der Beutel einen Schichtstoffverbund umfasst, der hydrophobe exponierte Oberflächen und ein abbaubares Mittelstück und Mittel zum Delaminieren des Beutels, wodurch das abbaubare Mittelstück exponiert und eine mühelose Spülung des Beutels nach der Verwendung unterstützt wird.

12. Stomabeutel nach einem der Ansprüche 1 bis 9, wobei der Beutel eine wegwerfbare oder abbaubare Auskleidung, die in einem hydrophoben Beutel eingeschlossen ist und Mittel zum bequemen Entfernen der Auskleidung zur Entsorgung umfasst.

13. Stomabeutel nach einem der vorhergehenden Ansprüche, der ferner eine verschließbare Austrittsöffnung und Mittel zum selektiven Freisetzen von angesammeltem Gas durch die Öffnung umfasst.

## Revendications

1. Poche de stomie ayant un orifice d'entrée conduisant à un espace de collecte de déchets défini par deux surfaces opposées, les surfaces opposées étant configurées pour se séparer lors de l'introduction de déchets à travers l'orifice d'entrée, **caractérisé par le fait qu'**elle comprend une lamelle ayant une surface de contact qui, lorsque la poche se trouve dans une configuration non utilisée, fait face à l'orifice d'entrée et qui, durant l'utilisation, lorsqu'un dépôt initial est fait à travers l'orifice d'entrée, collecte le dépôt et sous le poids du dépôt collecté tombe dans la poche séparant ainsi les surfaces opposées et ouvrant l'espace de collecte pour la réception d'autres dépôts.

2. Poche de stomie selon la revendication 1, dans laquelle une extrémité de la lamelle est soudée en un joint de soudure joignant les deux surfaces opposées au niveau d'une position distal relativement à l'emplacement de l'orifice d'entrée.

3. Poche de stomie selon la revendication 1 ou la revendication 2, dans laquelle la lamelle est dimensionnée pour s'étendre au-dessus de l'orifice d'entrée et à travers celui-ci de manière à être le premier point de contact d'un dépôt initial dans la poche.

4. Poche de stomie selon l'une quelconque des revendications précédentes, dans laquelle la lamelle est faite en un matériau souple et léger.

5. Poche de stomie selon la revendication 4, dans laquelle la lamelle comprend un film sélectionné parmi l'acétate de polyvinyle, l'EVA ou le polyéthylène.

6. Poche de stomie selon la revendication 4, dans laquelle la lamelle comprend un tissu non tissé adapté.

7. Poche de stomie selon l'une quelconque des revendications précédentes, dans laquelle l'aire de surface de la lamelle est comprise entre environ 60 % et 90 % de l'aire de surface d'une surface opposée.

8. Poche de stomie selon l'une quelconque des revendications précédentes, dans laquelle la lamelle a une taille et une forme de périmètre généralement similaires à celles de la poche mais avec une marge supprimée.

9. Poche de stomie selon l'une quelconque des revendications précédentes, dans laquelle la surface faisant face à l'orifice d'entrée est dotée d'un pli permettant ainsi une expansion supplémentaire de l'espace de collecte lorsque d'autres dépôts sont reçus dans la poche.

10. Poche de stomie selon la revendication 8, dans laquelle le pli est sélectionné parmi un pli unique, un double pli ou un pli creux.

11. Poche de stomie selon l'une quelconque des revendications précédentes, dans laquelle la poche comprend un stratifié qui a des surfaces hydrophobes exposées et un coeur dégradable et un moyen de délamination de la poche pour ainsi exposer le coeur dégradable et faciliter un rinçage rapide de la poche après l'utilisation.

12. Poche de stomie selon l'une quelconque des revendications 1 à 9, dans laquelle la poche comprend un revêtement jetable ou dégradable enfermé dans une poche hydrophobe et un moyen permettant le retrait pratique du revêtement pour son élimination.

13. Poche de stomie selon l'une quelconque des revendications précédentes, comprenant en outre une ouverture de sortie refermable et un moyen pour sélectivement libérer, à travers l'ouverture, le gaz accumulé.
